# EUROPEAN PATENT APPLICATION

(11) **EP 3 211 590 A1**
(43) Date of publication of application: **30.08.2017**
(21) Application number: 15872895.6
(22) Date of filing: 17.12.2015
(51) Int. Cl.: G06Q 50/22, A61B 5/00

(54) **LIVING-HABIT IMPROVEMENT DEVICE, LIVING-HABIT IMPROVEMENT METHOD, AND LIVING-HABIT IMPROVEMENT SYSTEM**

(30) Priority: 25.12.2014 JP 2014262336
(71) Applicant: Omron Corporation, Kyoto-shi, Kyoto 600-8530 (JP)
(72) Inventor: HASHIZAKI Masanori, Kyoto-shi Kyoto 600-8530 (JP); YAMAMOTO Junya, Kyoto-shi Kyoto 600-8530 (JP); NAKAJIMA Hiroshi, Kyoto-shi Kyoto 600-8530 (JP)
(74) Representative: Horn Kleimann Waitzhofer Patentanwälte PartG mbB
(86) International application number: PCT/JP2015/085368
(87) International publication number: WO 2016/104326

(57) **Abstract**

A lifestyle improvement device includes: a lifestyle pattern generator configured to generate a lifestyle pattern based on biological information that is of information about physiology caused by object person's physical activity and behavior information that is of object person's dynamic information; and a lifestyle predictor configured to predict the biological information about the object person based on the lifestyle pattern generated with the lifestyle pattern generator.

## Description

### TECHNICAL FIELD

The present invention relates to a lifestyle improvement device, a lifestyle improvement method in which the lifestyle improvement device is used, and a lifestyle improvement system in which the lifestyle improvement device is used. Hereinafter, a person who works on lifestyle management is generally referred to as an object person.

### BACKGROUND ART

Recently, consciousness for prevention of a disease largely caused by a lifestyle has been improved. A person who already has a disease and a person who possibly has a disease tend to be required to improve their lifestyle. Many healthy persons live with a strong consciousness of keeping their health up.

Because of this tendency, there is a demand for a technology and service of health management.

Patent Document 1 discloses a technology of classifying a lifestyle pattern.

Patent Documents 2 and 3 disclose a technology of managing a sleep state.

Thus, in the technology of Patent Documents 2 and 3, a behavior pattern such as exercise and a sleep state are linked together, and a recommended number of steps for getting good sleep, a time in bed, or a sleep time is provided.

### PRIOR ART DOCUMENTS

### PATENT DOCUMENTS

Patent Document 1: Japanese Patent No. 5466713
Patent Document 2: Japanese Unexamined Patent Publication No. 2014-30494
Patent Document 3: Japanese Patent No. 4192127

### SUMMARY OF THE INVENTION

### PROBLEMS TO BE SOLVED BY THE INVENTION

In the conventional technology associated with the sleep improvement, the behavior pattern such as the exercise of each person and the sleep state are analyzed while linked together, and a recommended behavior is proposed to get good sleep. That is, an evaluation is made based on only the sleep and the exercise, what is called behavior information.

Generally, in the case that an object person does not recognize a difference between a current pattern and an ideal pattern, because the object person insufficiently understands an issue about himself/herself, the object person does not always accept the recommended behavior. Even if the object person conforms to the recommended behavior, sometimes the object person hardly keeps consciousness of continuing the recommended behavior.

Additionally, prediction what kind of results the current lifestyle brings in future has been insufficiently made in the conventional technology. This is because a database in which information about the lifestyle is stored insufficiently exists.

An object of the present invention is to provide a lifestyle improvement device and a lifestyle improvement method, for being able to accurately recognize an object person's current situation not based on management based on only the behavior information such as the sleep and the exercise, but based on various pieces of information, such as biological information such as a body weight and a blood pressure and the behavior information such as the sleep and the exercise, which indicate the object person's state.

Another object of the present invention is to provide a lifestyle improvement device, a lifestyle improvement method, and a lifestyle improvement system, for being able to clarify an object person's current issue by accurately recognizing the object person's current situation, and transmit useful information about the lifestyle improvement to the object person by predicting the biological information in consideration of the object person's issue.

### MEANS FOR SOLVING THE PROBLEM

According to one aspect of the present invention, a lifestyle improvement device includes: a lifestyle pattern generator configured to generate a lifestyle pattern based on biological information that is of information about an object person's body and behavior information that is of information about an object person's behavior; and a lifestyle predictor configured to predict the biological information about the object person based on the lifestyle pattern generated with the lifestyle pattern generator.

According to another aspect of the present invention, a lifestyle improvement method includes: generating a lifestyle pattern based on biological information that is of information about physiology caused by object person's physical activity and behavior information that is of object person's dynamic information; and predicting the biological information about the object person based on the generated lifestyle pattern.

According to another aspect of the present invention, a lifestyle improvement system includes: a biological information database configured to accumulate biological information that is of information about an object person's body; a behavior information database configured to accumulate behavior information that is of information about an object person's behavior; and a lifestyle pattern generator configured to generate a lifestyle pattern based on the stored biological information and the stored behavior information. A message is transmitted to the object person based on the generated lifestyle pattern.

### EFFECT OF THE INVENTION

In the lifestyle improvement device, lifestyle improvement method, and lifestyle improvement system of the present invention, the generated object person's lifestyle pattern is correlated with the biological information and the behavior information. Therefore, the object person's current situation can accurately be recognized and the object person's current issue can be clarified.

The object person's future biological information is predicted in consideration of the object person's current situation and issue, and the prediction of the object person's future biological information is useful to transmission of information about the lifestyle improvement to the object person.

### BRIEF DESCRIPTION OF THE DRAWINGS

Fig. 1 is a block diagram illustrating a configuration of a lifestyle improvement device according to an embodiment of the present invention.
Fig. 2 is a flowchart illustrating processing of the lifestyle improvement device of the embodiment.
Fig. 3 is a view illustrating a basic database of an object person in the embodiment.
Fig. 4 is a view illustrating body weight measurement history data of the object person in the embodiment.
Fig. 5 is a view illustrating blood pressure measurement history data of the object person in the embodiment.
Fig. 6 is a view illustrating sleep information measurement history data of the object person in the embodiment.
Fig. 7 is a view illustrating behavior information measurement history data of the object person in the embodiment.
Fig. 8 is a view illustrating the lifestyle improvement device of the embodiment, and is a view illustrating a time-series weight fluctuation.
Fig. 9 is a view illustrating the lifestyle improvement device of the embodiment, and is a view illustrating an average number of steps per hour in a 24-hour range with respect to a weight fluctuation in a weight loss period and a weight gain period.
Fig. 10 is a view illustrating the lifestyle improvement device of the embodiment, and is a view illustrating a time-in-bed probability in the 24-hour range with respect to the weight fluctuation in the weight loss period and the weight gain period.
Fig. 11 is a view illustrating the lifestyle improvement device of the embodiment, and is a view illustrating a blood pressure measurement probability in a range from 6:00 AM to 12:00 PM with respect to the weight fluctuation in the weight loss period and the weight gain period.
Fig. 12 is a view illustrating the lifestyle improvement device of the embodiment, and is a view illustrating a basal body temperature measurement probability in a range from 5:00 AM to 12:00 PM with respect to the weight fluctuation in the weight loss period and the weight gain period.
Fig. 13 is a view illustrating a lifestyle improvement device of another embodiment, and is a view illustrating a time-series weight fluctuation.

### MODE FOR CARRYING OUT THE INVENTION

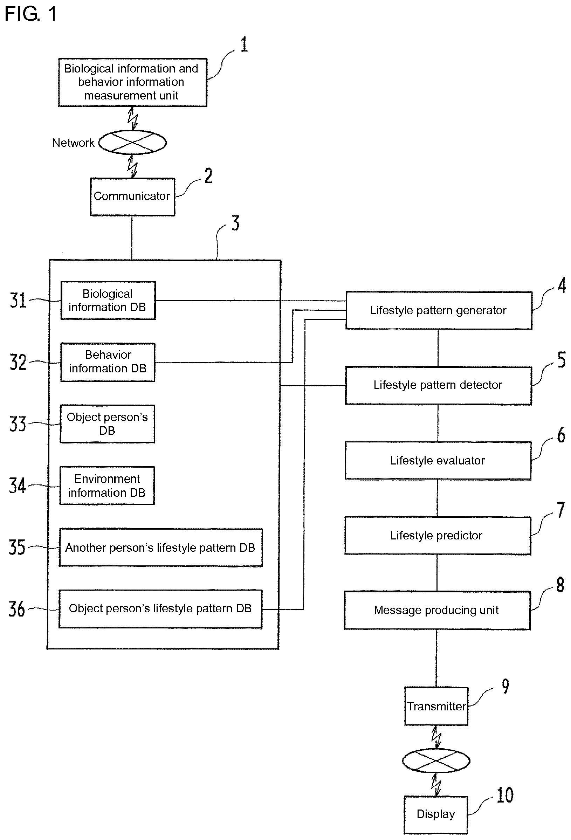

Fig. 1 is a block diagram illustrating a configuration of a lifestyle improvement device according to an embodiment.

The lifestyle improvement device of the embodiment includes a measurement unit 1 that obtains biological information and behavior information and a communicator 2 that receives data from the measurement unit 1, for example, a mobile terminal (not illustrated) through a network and transmits the data to a database 3 (to be described later).

The database 3 includes a biological information database 31, a behavior information database 32, an object person database 33, an environment information database 34, an another person's lifestyle pattern database 35, and an object person's lifestyle pattern database 36. Object person's biological information measured with a biological information measurement device is stored in the biological information database 31 together with a measurement history of the object person's biological information. Object person's behavior information measured with a behavior information measurement device is stored in the behavior information database 32 together with a measurement history of the object person's behavior information. Object person's basic information is stored in the object person database 33. Environment information is stored in the environment information database 34. A lifestyle pattern of another person except for the object person is stored in the another person's lifestyle pattern database 35. An object person's lifestyle pattern generated with a lifestyle pattern generator 4 (to be described later) is stored in the object person's lifestyle pattern database 36. A lifestyle pattern of a person who is an ideal person as another person may be included in the another person's lifestyle pattern database 35.

The lifestyle improvement device also includes a lifestyle pattern generator 4, a lifestyle pattern detector 5, a lifestyle evaluator 6, a lifestyle predictor 7, a message producing unit 8, a display 10, and a transmitter 9. The lifestyle pattern generator 4 generates the object person's lifestyle pattern based on the pieces of information stored in the biological information database 31 and behavior information database 32. The lifestyle pattern detector 5 detects a lifestyle pattern closest to a recent lifestyle pattern (including a current lifestyle pattern) from past lifestyle patterns of the object person. The lifestyle evaluator 6 compares the detected lifestyle pattern to a specific lifestyle pattern, extracts a difference between the detected lifestyle pattern to the specific lifestyle pattern, and extracts an issue of a current lifestyle pattern. The lifestyle predictor 7 predicts the biological information when the object person keeps the current lifestyle pattern up. The message producing unit 8 produces a message based on evaluation information obtained with the lifestyle evaluator 6 and prediction information obtained with the lifestyle predictor 7. The display 10 displays the message. The transmitter 9 transmits the message to the display 10.

As used herein, the biological information means information about an object person's body.

Specifically, examples of the biological information include, but not limited to, a body weight, a body fat percentage, a body fat level, a basal metabolism, a skeletal muscle ratio, a physical age, and a body mass index (BMI), which are measurable with a weight and body composition meter or a body weight meter, a maximal blood pressure and a minimal blood pressure, which are measurable with a sphygmomanometer, a subcutaneous fat thickness which is measurable with an adipometer, a body fat level which is measurable with a body fat meter, a heart rate which is measurable with a heart rate meter, and a body temperature including a basal body temperature which is measurable with a clinical thermometer.

The behavior information means information about an object person's behavior.

Specifically, examples of the behavior information include, but not limited to, a sleep time, a time in bed the object person goes to bed, and a wake-up time the object person goes out from bed, which are measurable with a sleep meter, a number of steps (a number of fast-walking steps and a number of steps in going up stairs may be distinguished) per unit time, time the biological information is measured, whether the biological information is measured, a consumed calorie, a fat burning amount, time a wearable device is put on or taken off, and time for which the wearable device is mounted, which are measurable with a pedometer or an activity amount meter, a duration, a number of steps, and a movement distance of jogging or the like, which are measurable with a life-log such as a wearable device, a mastication frequency which is measurable with a mastication frequency meter, and a movement distance which is measurable with a global positioning system (GPS).

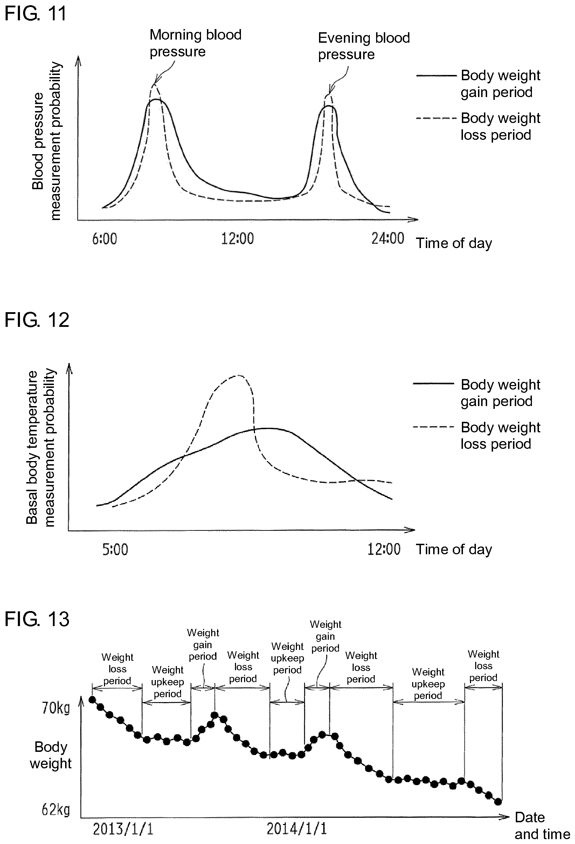

A measurement probability in Figs. 11 and 12 is an example of the behavior information.

Fig. 11 illustrates a blood pressure measurement probability in each of a body weight gain period and a body weight loss period of the object person. The blood pressure measurement probability means a ratio at which, based on time the blood pressure is measured, the blood pressure is measured at the time. It is assumed that the blood pressure is measured at specific times in the morning and evening. Specifically, it is assumed that the morning blood pressure measurement is performed within one hour after the wake-up time, and that the evening blood pressure measurement is performed immediately before the time in bed. Accordingly, a target time of the blood pressure measurement ranges from 6:00 AM to 12:00 AM.

Referring to Fig. 11, in the body weight loss period, because the measurement probability exhibits peaks in specific morning and evening time zones compared with the body weight gain period, it can be recognized that the object person performs the measurement with regularity, and that wake-up and going-to-bed rhythms are stable. That is, in the body weight loss period, it is considered that the object person has a high consciousness about the lifestyle, and that motivation is improved.

Fig. 12 illustrates a basal body temperature measurement probability in each of the body weight gain period and body weight loss period of the object person. The basal body temperature measurement probability means a ratio at which, based on time the basal body temperature is measured, the basal body temperature is measured at the time. The object person is recommended to measure the basal body temperature immediately after the object person wakes up and at an identical time as much as the object person can. In the example of Fig. 12, a measurement time of the basal body temperature ranges from 5:00 AM to 12:00 PM.

Referring to Fig. 12, in the body weight loss period, because the measurement probability exhibits peaks in a specific morning time zone compared with the body weight gain period, it can be recognized that the object person performs the measurement with regularity, and that the wake-up rhythm is stable. Even in the example of Fig. 12, in the body weight loss period, it is considered that the object person has a high consciousness about the lifestyle, and that motivation is improved.

The biological information measurement probability is applied as the behavior information to the lifestyle improvement device.

The lifestyle pattern is information indicating a lifestyle that is a daily routine behavior, and means an information aggregate, which is formed based on the biological information and the behavior information.

The measurement unit 1 includes the biological information measurement device and the behavior information measurement device. These measurement devices may be a measurement device that measures both the biological information and the behavior information.

For example, not only the sleep meter or the sphygmomanometer is used as the measurement device that obtains one of the biological information and the behavior information, but also the sleep meter or the sphygmomanometer is used as the measurement device that simultaneously obtains both the biological information and the behavior information.

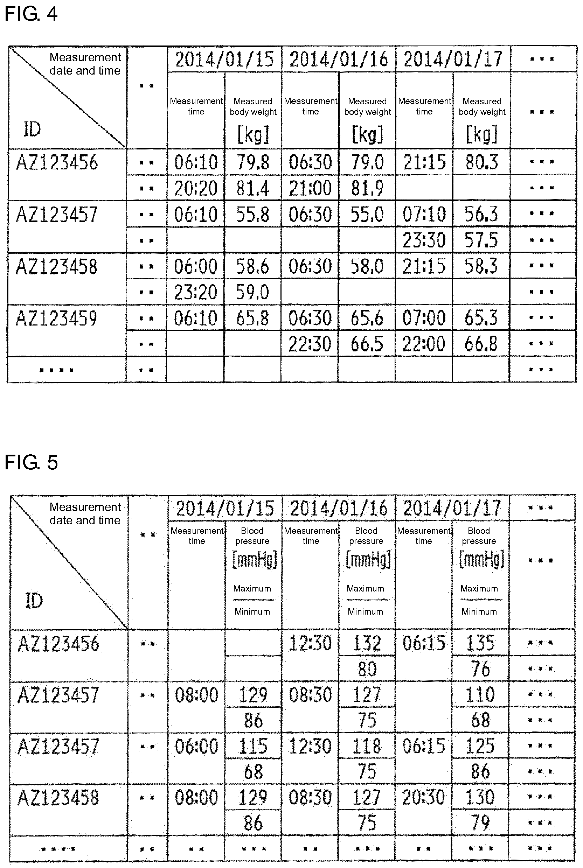

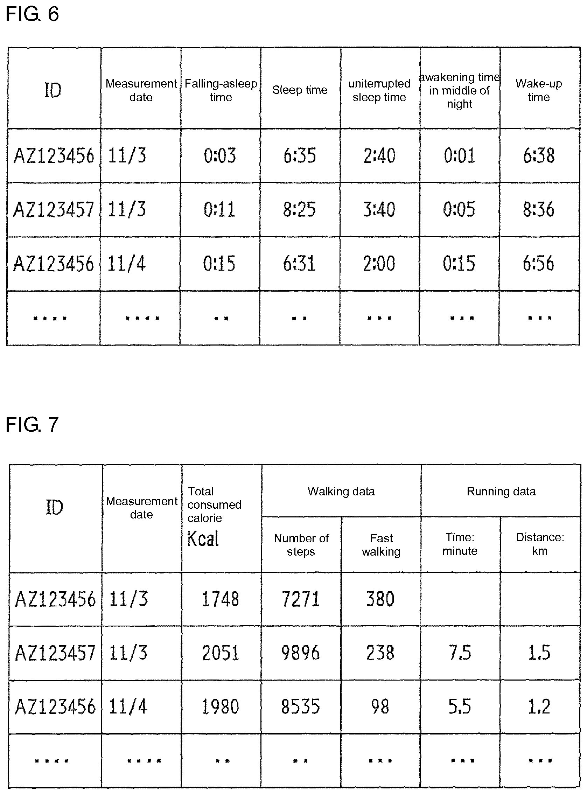

Specifically, as illustrated in Fig. 5, in performing the measurement, the sphygmomanometer obtains the maximal blood pressure and the minimal blood pressure as the biological information, and obtains the time the blood pressure is measured as the behavior information. As illustrated in Fig. 6, the sleep meter obtains the sleep time, the object person's uninterrupted sleep time, and the like as the biological information, and obtains the measurement date of the sleep meter, the wake-up time, and the like as the behavior information.

In addition to the biological information or behavior information measurement function, the biological information measurement device or behavior information measurement device includes a near-field radio communication function of transmitting a measurement result to a mobile terminal by near-field radio communication. Examples of the near-field radio communication function include, but not limited to, near field communication (NFC), communication by Felica (registered trademark), universal serial bus (USB) communication, and communication by Bluetooth (registered trademark).

The mobile terminal (not illustrated) includes the near-field radio communication function of conducting the near-field radio communication with the biological information measurement device or the behavior information measurement device and a network communication function of conducting communication through a network such as the Internet. Specifically, the mobile terminal transmits data, in which an identification code (ID) is added to the measurement data measured with the biological information measurement device or the behavior information measurement device, to the communicator 2. In this kind of mobile terminal, there is an already-commoditized smartphone. Alternatively, a tablet computer or a wearable computer may be used. The mobile terminal can also be substituted for a personal computer. Desirably the object person individually owns the mobile terminal or the substitute for the mobile terminal.

The communicator 2 receives the ID-added measurement data, which is transmitted from the mobile terminal. The measurement data is stored in the biological information database 31 and the behavior information database 32 through the communicator 2.

A value measured with each measurement device and a measurement date and time (date and time) are stored as the measurement history in biological information database 31 and the behavior information database 32. For example, Fig. 4 illustrates body weight measurement history data, and Fig. 5 illustrates blood pressure measurement history data. The measurement date and time of the body weight and the body weight measured at the date and time are stored every object person having a different ID in the databases 31, 32 in which the pieces of measurement history data are stored.

As illustrated in Figs. 4 and 5, an area where the measurement data is stored is provided in the database, but the measurement data does not exist in an area where the measurement data is not stored. The measurement data depends on each object person, and frequently the identical object person has different data in time series.

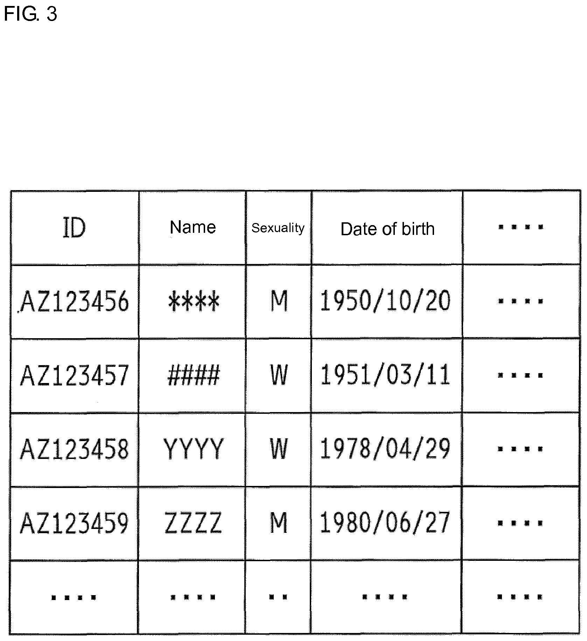

As illustrated in Fig. 3, examples of the object person's basic information include, but not limited to, the ID used to identify the object person and a name, sexuality, and a date of birth of the object person as the object person's basic information.

Examples of the environment information include information, which is obtained with a system that obtains external environment information, and information, which is obtained with a device that obtains indoor environment information. Examples of the environment information include, but not limited to, temperature, humidity, and illuminance, which fluctuate depending on a weather or a season in a period in which the biological information or behavior information is measured.

An another person's lifestyle pattern is stored in the another person's lifestyle pattern database 35. Specifically, the lifestyle pattern including the basic information such as sexuality and age, the biological information such as a body height and a body weight, and the behavior information such as exercise is previously produced.

By previously obtaining the lifestyle pattern of the ideal person such as an athlete, the lifestyle pattern of the ideal person may be stored in the another person's lifestyle pattern database 35.

Any measurement device associated with a target item that the object person wants to improve may be used as the biological information measurement device and behavior information measurement device, which are used in the lifestyle improvement system. In the case that a plurality of measurement devices are used, preferably the obtained data is rich, and the state of the object person can more specifically be recognized.

For example, in the case that the object person wants to lose weight, not only the body weight meter and the pedometer are used, but also a multilateral analysis may be performed by analyzing a correlation between the sleep and the body weight using the sleep meter.

The lifestyle pattern generator 4 generates the lifestyle pattern based on the state indicated by the object person's biological information and the behavior information.

The behavior information cited as a constituent for generating the lifestyle pattern is linked to the state indicated by the following classified biological information.

The state indicated by the biological information can be classified by a method for making a determination from a time-series context. For example, the state is determined to be one of "improvement", "deterioration", and "upkeep" in time series, and classified into "improvement", "deterioration", and "upkeep". The state is classified by analyzing whether a value of the state is increased, decreased, or unchanged compared with a previous value. Alternatively, a method for analyzing whether a gradient of a line by using linear regression is larger than or equal to a given value or less than a given value in a certain period, may be adopted.

The method for classifying the biological information in a certain period into the states is not limited to the method for determining the state from the time-series context, but a method for determining the state by comparison between a value of a stationary point value and a criteria value may be adopted. For example, the state is classified by a determination whether the object person is sick. The comparison to the criteria value such as a high blood pressure criteria (maximal blood pressure/minimal blood pressure) of 135/85 [mmHg] in terms of home blood pressure and a fatness criteria BMI of 25 or more can be cited as an example, and the state can be classified by the comparison.

A method in which the method for determining the state from the time-series context and the state classified using the criteria value are combined with each other may be adopted as the method for classifying the state indicated by the biological information.

For example, the state of change is classified into "weight loss", "weight gain", and "weight upkeep" in terms of the body weight, classified into "slimness", "ordinary level", "fatness" in terms of the criteria value for fatness, and newly classified by a combination of the classifications.

As to the blood pressure, the state of change is classified into "blood pressure fall", "blood pressure rise", and "blood pressure upkeep", classified into "high blood pressure", "high normal blood pressure", and "normal value" in terms of the criteria value for high blood pressures, and newly classified by a combination of the classifications.

A specific example of a method for generating the lifestyle pattern in the lifestyle pattern generator 4 will be described below.

An object person's body weight data is obtained from the biological information database 31, and a sleep data and an exercise data are obtained from the behavior information database 32.

Whether the object person's past data is sufficiently stored is determined in obtaining the pieces of data. At this point, a certain amount of object person's data is required. For example, a data amount for one month, three months, or one year is enough for the obtainment. The object person's data itself is used when the data is sufficiently stored.

On the other hand, when the data is insufficiently stored, an another person's lifestyle pattern similar to that of the object person is extracted from the another person's lifestyle pattern database 35, and used as the object person's data. Examples of requirements for the similarity include the basic information such as the sexuality and the age, the biological information such as the body height, and the sleep information such as the sleep data. The data of a person whose requirements are similar to those of the object person is selected as the object person's data.

At a point of time the object person's data is sufficiently stored, the processing is performed while the another person's data is not used, but the another person's data is switched to the object person's data.

A daily 24-hour lifestyle pattern is produced based on the obtained past sleep data and exercise data of the object person or the past sleep data and exercise data of the person regarded as the object person.

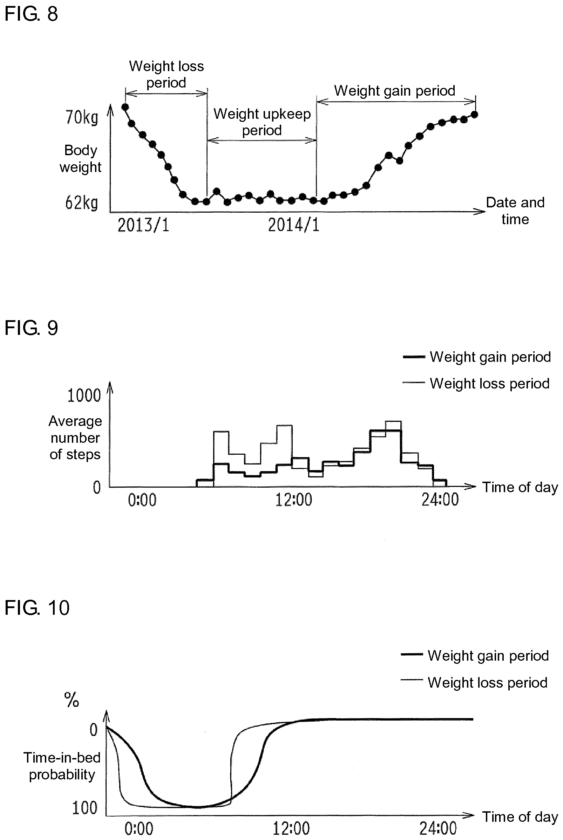

Then, among the pieces of biological information about the object person, the body weight is classified in each given period, for example, in each one week, into "weight upkeep state" in which the body weight fluctuates by ±2%, "weight gain state" in which the body weight fluctuates by +2 to +5%, and "weight loss state" in which the body weight fluctuates by -2 to -5%. Fig. 8 illustrates a time-series weight fluctuation of the object person, and illustrates the state of the body weight in each period, which is classified by the above classification method into the "weight loss period", "weight upkeep period", and "weight gain period".

Then, similarly to the daily 24-hour lifestyle pattern based on the above past sleep data and exercise data, a recent 24-hour lifestyle pattern is produced based on the recent (including the current sleep data and exercise data) sleep data and exercise data of the object person. There is no particular limitation to a range of the period called "recent", but the range may be one day or one week.

Then, a time-in-bed probability and an average number of steps, which is of an average exercise amount, are calculated in each of the "weight upkeep period", "weight gain period", and "weight loss period" of the above state of the body weight from the sleep data and exercise data, which are measured in each period.

Fig. 9 illustrates the average number of steps per hour in a 24-hour range in each of the "weight loss period" and "weight gain period" of the state of the body weight.

Fig. 10 illustrates the time-in-bed probability in a 24-hour range in each of the "weight loss period" and "weight gain period" of the state of the body weight.

As illustrated in Figs. 9 and 10, the body weight data (biological information) is linked to the number of steps data (behavior information) and the sleep data (behavior information), which generates the lifestyle pattern in each of the "weight loss period" and "weight gain period".

The generated lifestyle pattern is stored in the object person's lifestyle pattern database 36.

The lifestyle pattern detector 5 detects the lifestyle pattern closest to the recent (including the current lifestyle pattern) lifestyle pattern of the object person from the past object person's lifestyle pattern stored in the object person's lifestyle pattern database 36. Specifically, for example, the lifestyle pattern linked to the behavior information such as the closest daily average number of steps is detected.

Methods such as a correlation, machine learning, a recommendation algorithm, and a statistical technique can be adopted when the past object person's lifestyle pattern and the recent (including the current lifestyle pattern) lifestyle pattern of the object person are compared to each other.

The lifestyle evaluator 6 extracts a difference between the lifestyle pattern closest to the recent (including the current lifestyle pattern) lifestyle pattern detected with the lifestyle pattern detector 5 and the lifestyle pattern in the "weight loss period" or "weight upkeep period". For example, the difference between the two lifestyle patterns is obtained by comparison of pieces of sleep data such as a falling-asleep time, a wake-up time, a sleep time, a uninterrupted sleep time, and an awakening time in middle of night in Fig. 6 or pieces of exercise data such as walking data in Fig. 7 to each other. The issue of the recent lifestyle pattern is extracted based on the difference.

The lifestyle predictor 7 predicts a change in the body weight of the object person in future, for example, one week, one month, or one year based on the past lifestyle pattern and the past lifestyle pattern closest to the recent lifestyle pattern.

Fig. 13 illustrates a time-series weight fluctuation in which the data is obtained in a period longer than that in Fig. 8. In the case that the data in Fig. 13 is used, understanding of the current state and the prediction of the future body weight can be performed from the viewpoint of an appearance pattern of the state and a length of the appearance pattern. In the example of Fig. 13, in the case that the "weight loss period", "weight upkeep period", and "weight gain period" are easily repeated in the appearance pattern, or in the case that the appearance pattern has the short "weight upkeep period", it is predicted that regaining weight is easy to occur. On the other hand, in the case that the appearance pattern has the long "weight upkeep period" it is predicted that regaining weight does not occur, but the "weight upkeep period" is easily transferred to the "weight loss period".

The use of the data changing in time series can understand the current state, and accurately predict the future state.

During the prediction, an influence of the pieces of environment information stored in the environment information database 34, for example, temperature, humidity, and illuminance, on the biological information are individually evaluated in addition to the sleep data or exercise data, which are stored by the object person. Therefore, an influence of the fluctuation of the biological information on the lifestyle and the environment can also be calculated. The fluctuation of the biological information can be predicted based on a calculation result.

The message producing unit 8 produces the issue extracted with the lifestyle evaluator 6 and the prediction information predicted with the lifestyle predictor 7 as the message. Desirably the message includes information about which the improvement effect can be expected most for the object person or information in which the biological information is not deteriorated.

In the case that the past lifestyle pattern close to the recent lifestyle pattern does not exist, this point is included in the prediction message. In such cases, for example, such a large change that the data does not exist within past two years is recognized, and the message that the recent lifestyle pattern becomes the worst in recent years or the message that the recent lifestyle pattern becomes the best in recent years is output.

The transmitter 9 transmits the message and/or prediction message indicating the issue to the display 10 through the network.

Various transmitted messages are displayed on the display 10, which allows the object person to recognize the messages. Devices such as a mobile terminal, a smartphone, a tablet computer, and a personal computer are appropriately used as the display 10.

In the embodiment, by way of example, the body weight is classified into the "weight loss period", "weight gain period", and "weight upkeep period" as the state of the biological information. Alternatively, the blood pressure may be classified into "blood pressure falling period", "blood pressure rising period", and "weight upkeep period", or the basal body temperature may be classified into a menstrual cycle, an ovulation day, and a stable period or a unstable period of the number of a low temperature phase and a high temperature phase, which can be appropriately selected.

### [Effect of lifestyle improvement device]

In the lifestyle improvement device of the embodiment having the above configuration, the lifestyle pattern generator generates the lifestyle pattern by the combination of the biological information and the behavior information linked to the biological information. Therefore, the object person's lifestyle pattern conforms to the object person's actual state. The difference between the recent (including the current lifestyle pattern) lifestyle pattern of the object person and the past object person's lifestyle pattern can clearly be understood by the comparison between the recent (including the current lifestyle pattern) lifestyle pattern of the object person and the past object person's lifestyle pattern.

Conventionally, the prediction what kind of results the recent lifestyle brings in future is not made. On the other hand, in the lifestyle improvement device, the biological information such as the future body weight of the object person can be predicted, and the lifestyle improvement information can be provided to the object person based on the prediction.

In the prediction processing, the fluctuation of the biological information can be predicted in consideration of the influence of the environment information on the biological information, for example, the fluctuation of the body weight or blood pressure can be predicted. Resultantly, the object person is highly satisfied with the prediction message, and support can efficiently be provided for the purpose of the improvement of the lifestyle and the environment.

As described above, the object person can obtain the recent issue of the object person and the information about the prediction in future. Therefore, when the object person performs the improvement of the lifestyle and the environment, the object person can be expected to sufficiently understand necessity or importance of the improvement of the lifestyle and the environment, perform necessary action with satisfaction, and keep up the motivation to continue the action.

### <Lifestyle evaluator according to another embodiment of the present invention>

In the configuration of the embodiment, the object person's recent (including the current lifestyle pattern) lifestyle pattern is compared to the object person's past lifestyle pattern closest to the recent lifestyle pattern. In another embodiment, an object person's ideal person previously stored in the another person's lifestyle pattern database 35, for example, a lifestyle pattern of an athlete is selected instead of the object person's past lifestyle pattern closest to the recent (including the current lifestyle pattern) lifestyle pattern, a difference between the lifestyle pattern of the athlete and the lifestyle pattern of the object person can be understood by the comparison of the lifestyle pattern of the athlete to the lifestyle pattern of the object person.

The desirable state of the object person's lifestyle pattern can easily be realized by focusing on the athlete admired by the object person or the lifestyle pattern of the target athlete. These points can have a good influence on object person's mentality, and keep up the object person's motivation to keep up or improve the object person's health.

### [Lifestyle improvement method]

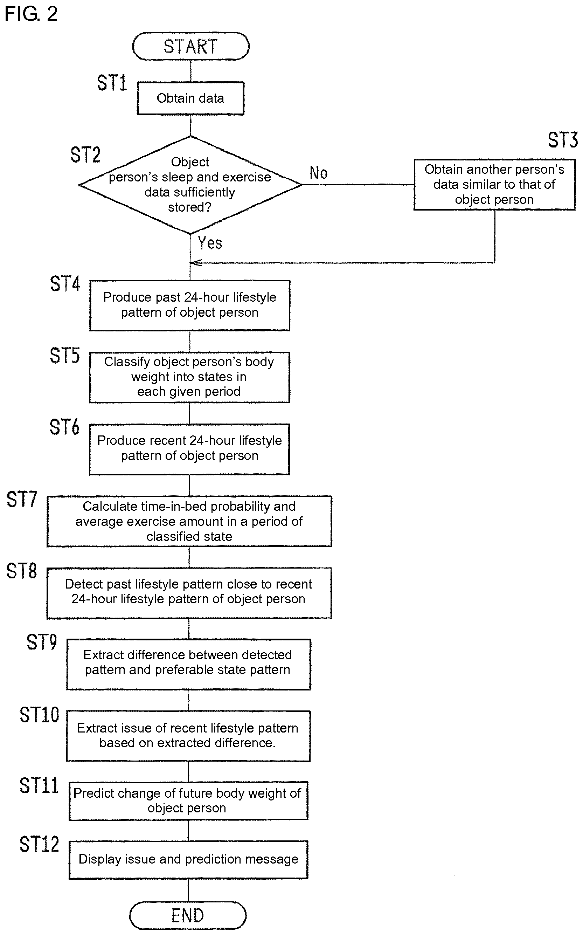

A lifestyle improvement method of the embodiment will be described below with reference to a flowchart illustrating processing of the lifestyle improvement device of the embodiment in Fig. 2.

In the embodiment, the body weight data is used as the biological information, and the sleep data and the exercise data are used as the behavior information.

The object person's body weight data is obtained from the biological information database 31, and the sleep data and exercise data are obtained from the behavior information database 32 (ST1).

Whether the object person's past data is sufficiently stored is determined in obtaining the pieces of data (ST2). A certain amount of object person's data is required. For example, a data amount for one month, three months, or one year is enough for the obtainment. When the data is sufficiently stored, processing in step 4 (to be described later) is performed using the object person's data (ST4).

On the other hand, when the data is insufficiently stored, an another person's lifestyle pattern similar to that of the object person is extracted from the another person's lifestyle pattern database 35, and used as the object person's data. Examples of the requirements for the similarity include the basic information such as the sexuality and the age, the biological information such as the body height, and the sleep information such as the sleep data. In this case, the data of the person whose requirements are similar to those of the object person is selected as the object person's data (ST3), and the processing in step 4 (ST4) is performed.

At a point of time the object person's data is sufficiently stored, the processing is performed while the another person's data is not used, but the another person's data is switched to the object person's data.

In step 4 (ST4), the daily 24-hour lifestyle pattern is produced based on the obtained past sleep data and exercise data of the object person or the past sleep data and exercise data of the person regarded as the object person.

Then, among the pieces of biological information about the object person, the body weight is classified in each given period, for example, in each one week, into "weight upkeep state" in which the body weight fluctuates by ±2%, "weight gain state" in which the body weight fluctuates by +2 to +5%, and "weight loss state" in which the body weight fluctuates by -2 to -5%. A flag is set to the data such that the "weight upkeep state", the "weight gain state", and the "weight loss state" can be distinguished from one another (ST5).

Then, similarly to the past daily 24-hour lifestyle pattern of the object person in step (ST4), a recent 24-hour lifestyle pattern of the object person is produced based on the recent (including the current sleep data and exercise data) sleep data and exercise data of the object person. There is no particular limitation to the range of the period called "recent", but the range may be one day or one week (ST6).

Then, the time-in-bed probability and the average number of steps are calculated in each of the "weight upkeep state", "weight gain state", and "weight loss state" of the above state of the body weight from the sleep data and exercise data, which are measured in each period (ST7).

The past object person's lifestyle pattern is compared to the recent (including the current lifestyle pattern) lifestyle pattern of the object person, and the lifestyle pattern closest to the recent (including the current lifestyle pattern) lifestyle pattern is detected (ST8).

The difference between the lifestyle pattern closest to the detected recent (including the current lifestyle pattern) lifestyle pattern and the lifestyle pattern in the state of the preferable body weight, for example, the lifestyle pattern in the "weight loss state" or the lifestyle pattern in the "weight upkeep state" is extracted (ST9).

The issue of the recent lifestyle pattern is extracted based on the difference (ST10).

The change in body weight of the object person is predicted in future, for example, one week, one month, or one year based on the past lifestyle pattern and the past lifestyle pattern closest to the recent lifestyle pattern (ST11).

The issue extracted in step (ST10) and a prediction result obtained in step (ST11) are produced and displayed as a message (ST12).

Because the effect of the lifestyle improvement method is similar to that of the above lifestyle improvement device, the description is omitted.

This application claims priority to Japanese Patent Application No. 2014-262336 filed with the Japan Patent Office on December 25, 2014, the entire contents of which are incorporated herein by reference. The cited documents are incorporated herein by reference in its entirety.

### DESCRIPTION OF SYMBOLS

- 3: database
- 31: biological information database
- 32: behavior information database
- 33: object person's database
- 34: environment information database
- 35: another person's lifestyle pattern database
- 36: object person's lifestyle pattern database
- 4: lifestyle pattern generator
- 5: lifestyle pattern detector
- 6: lifestyle evaluator
- 7: lifestyle predictor
- 8: message producing unit

## Claims

1. A lifestyle improvement device comprising:
a lifestyle pattern generator configured to generate a lifestyle pattern based on biological information that is of information about an object person's body and behavior information that is of information about an object person's behavior; and
a lifestyle predictor configured to predict the biological information about the object person based on the lifestyle pattern generated with the lifestyle pattern generator.

2. The lifestyle improvement device according to claim 1, wherein the lifestyle pattern generator classifies past biological information about the object person into states indicated by the past biological information, and generates the lifestyle pattern based on the behavior information in each state.

3. The lifestyle improvement device according to claim 1 or 2, wherein the state indicated by the biological information is classified based on at least one of a criteria value, which is previously set according to a type of the biological information, and a time-series change of the state.

4. The lifestyle improvement device according to claim 1 or 2, further comprising:
a memory in which the lifestyle pattern generated with the lifestyle pattern generator is stored; and
a lifestyle pattern detector configured to detect one lifestyle pattern from the lifestyle patterns stored in the memory.

5. The lifestyle improvement device according to claim 4, wherein the lifestyle pattern detector detects the lifestyle pattern based on the behavior information.

6. The lifestyle improvement device according to claim 4, wherein the lifestyle pattern detector detects a lifestyle pattern closest to a recent lifestyle pattern (including a current lifestyle pattern) of the object person from the past lifestyle patterns of the object person being stored in the memory.

7. The lifestyle improvement device according to claim 6, further comprising a lifestyle evaluator configured to recognize the detected past lifestyle pattern closest to the recent lifestyle pattern (including the current lifestyle pattern) of the object person as the recent lifestyle pattern of the object person, compare the recent lifestyle pattern to a specific lifestyle pattern, and extract an issue of a recent (including a current lifestyle) lifestyle of the object person.

8. The lifestyle improvement device according to claim 1 or 2, further comprising a lifestyle predictor configured to predict future biological information when the object person keeps a recent (including a current lifestyle pattern) lifestyle pattern up.

9. The lifestyle improvement device according to claim 8, wherein the lifestyle predictor predicts, when the object person keeps the recent (including the current lifestyle pattern) lifestyle pattern up, the future biological information based on an appearance pattern of the state indicated by the biological information and a duration for which the appearance pattern appears.

10. The lifestyle improvement device according to claim 7, further comprising a message producing unit configured to produce a message including information for improving or keeping up the object person's lifestyle based on the issue extracted with the lifestyle evaluator and prediction information predicted with the lifestyle evaluation predictor.

11. A lifestyle improvement method comprising:
generating a lifestyle pattern based on biological information that is of information about an object person's body and behavior information that is of information about an object person's behavior; and
predicting the biological information about the object person based on the generated lifestyle pattern.

12. The lifestyle improvement method according to claim 11, further comprising
classifying past specific biological information about the object person into states indicated by the past specific biological information; and
generating the lifestyle pattern based on the behavior information in each state.

13. The lifestyle improvement method according to claim 11 or 12, further comprising
classifying the state indicated by the biological information based on at least one of a criteria value, which is previously set according to a type of the biological information, and a time-series change of the state.

14. A lifestyle improvement system comprising:
a biological information database configured to store biological information that is of information about an object person's body;
a behavior information database configured to store behavior information that is of information about an object person's behavior; and
a lifestyle pattern generator configured to generate a lifestyle pattern based on the stored biological information and the stored behavior information,
wherein a message is transmitted to the object person based on the generated lifestyle pattern.
